# EUROPÄISCHE PATENTSCHRIFT

(11) **EP 0 270 987 B1**
(45) Veröffentlichungstag und Bekanntmachung des Hinweises auf die Patenterteilung: **13.03.1996**
(21) Anmeldenummer: 87117794.5
(22) Anmeldetag: 02.12.1987
(51) Int. Cl.: C08G 63/90, A61L 31/00, A61L 27/00, A61K 9/22, A61K 47/00

(54) **Verfahren zur Herstellung katalysatorfreier resorbierbarer Homopolymere und Copolymere.**
Process for preparation of catalyst-free resorbable homopolymers and copolymers.
Procédé de préparation de homopolymères et copolymères résorbables exempts de catalyseur.

(30) Priorität: 06.12.1986 DE 3641692
(43) Veröffentlichungstag der Anmeldung: 15.06.1988
(73) Patentinhaber: BOEHRINGER INGELHEIM KG, D-55216 Ingelheim (DE); BOEHRINGER INGELHEIM INTERNATIONAL GmbH, D-55216 Ingelheim am Rhein (DE)
(72) Erfinder: Bendix, Dieter, Dr. Dipl.-Chem., D-6507 Ingelheim am Rhein (DE); Entenmann, Günther, Dr. Dipl.-Chem., D-6507 Ingelheim (DE)

(56) Entgegenhaltungen:
- CH-A- 542 198
- GB-A- 1 193 240
- US-A- 3 629 202
- US-A- 3 797 499
- US-A- 3 991 766
- Encyclopedia of Polymer Science and Engineering, vol.12,1988,page 7
- Encyclopedia of Polymer Science and Engineering, vol.2,1985,page 230-232.

## Beschreibung

Die Vorliegende Erfindung betrifft ein Verfahren zur Herstellung katalysatorfreier resorbierbare Homopolymere und Copolymere und Verfahren zu ihrer Herstellung.

In den letzten Jahren hat das Interesse an der Verwendung resorbierbarer Polyester sehr stark zugenommen; so z.B. im Bereich der Chirurgie als Nahtmaterial oder Klammern, im Bereich der Osteosynthese oder als Wirkstoffträger mit verzögerter kontrollierter Wirkstofffreigabe. Wie aus zahlreichen Publikationen bekannt ist, liegt der große Vorteil resorbierbarer Polyester, insbesondere derer auf der Basis von Milch- oder Glykolsäure, in der Tatsache, daß sie im menschlichen oder tierischen Gewebe vollständig zu körpereigenen Verbindungen abgebaut werden. Diese Abbauprodukte gelangen in den normalen biochemischen Stoffwechsel und werden letztlich zu Wasser und Kohlendioxid metabolisiert. Nachteilig bei der Verwendung resorbierbarer Homopolymere und Copolymere erwies sich der Gehalt an Katalysator. Bei der Bioresorption der Polymere bleibt eine endliche Menge an Katalysator im Gewebe, da der Katalysator hier nicht abgebaut werden kann. Dadurch kann es im Gewebe zu Reizungen, je nach Katalysator sogar zu Intoxikationen kommen. Weiterhin kann der im Homopolymeren oder Copolymeren verbleibende Katalysator auch zu Umesterungen oder sogar zum unkontrollierten Abbau des Polymeren führen und damit beispielsweise zu einer nicht vorhersehbaren Freisetzungsrate eines Wirkstoffs aus einer Retardform. Unkontrollierter Abbau kann bei der Osteosynthese auch zum Bruch des Implantats führen.

Katalysatorfreie Copolymere auf der Basis von Milchsäure- und Glykolsäure sind bekannt. Durch direkte Kondensation von Milch- und Glykolsäure oder aber durch Cokondensation dieser beiden Verbindungen können niedermolekulare Polymere, vorzugsweise mit einem Molekulargewicht von 2000 his 4000 erhalten werden. Versuche, durch direkte Kondensation hohe Molekulargewichte zu erreichen, führten dagegen zur Abspaltung von Lactid oder Glycolid. Eine mögliche Ursache hierfür ist, daß zur Abtrennung des letztlich noch in Spuren vorhandenen Kondensationswassers der Polyesterbildung Druck und Temperatur so eingestellt werden müssen, daß hierbei die Reaktionsbedingungen der Lactid- bzw. Glycolidsynthese erreicht werden, so daß zumindest teilweise eine Depolymerisation erfolgt.

Aus der Europäischen Patentschrift 26 599 ist die Verwendung von stark sauren Ionenaustauschern zur Herstellung eines katalysatorfreien Copolymerisats aus Glycolid und Lactid bekannt. Nach dem dort beschriebenen Verfahren können Copolymere mit einem Molekulargewicht von 6000 bis maximal 35 000 hergestellt werden.

Weiterhin ist aus der europäischen Patentanmeldung 171 907 bekannt, Copolymerisate aus Milch- und Glykolsäure durch "dehydration polycondensation" mit Molekulargewichten bis ca. 20 700 herzustellen.

Wie im Verlauf der letzten Jahre gezeigt werden konnte, ist die Verwendung von resorbierbaren Polyestern sehr stark mit den chemischen und physikalischen Eigenschaften dieser Polymere verknüpft. Durch Variation der Zusammensetzung der Comonomere, der Polymerisationsverfahren und Auswahl geeigneter Katalysatoren war es möglich, entsprechend ihrer beabsichtigten Verwendung (z.B. Nahtmaterial, resorbierbares oder teilresorbierbares osteosynthetisches Material, galenische Darreichungsformen) angepaßte Polymere herzustellen, die jedoch endliche Mengen an Katalysator enthalten.

Aus dem Stand der Technik sind bislang lediglich katalysatorfreie Copolymere aus Lactid und Glycolid bekannt, die ein mittleres Molekulargewicht bis maximal 35 000 aufweisen. Dies bedeutet aber eine wesentliche Einschränkung ihrer Verwendung, da es in vielen Fällen erforderlich ist, Polymere mit höheren Molekulargewichten einzusetzen.

Verfahren zur Herstellung katalysatorfreier Polymere sind bereits aus dem Stand der Technik bekannt. - So offenbart die CH-A-0542198 ein Verfahren zur Herstellung von ß-Glykolid und dessen Verwendung. Das in dieser Offenlegungsschrift beschriebene Verfahren ist jedoch nicht dazu geeignet, um Polymere zur Verfügung zu stellen, die für die Herstellung von Nahtmaterial oder Implantaten geeignet sind. Der Grund hierfür ist in der atmosphärischen Feuchtigkeit zu sehen, die ein Zustandekommen hoher Molekulargewichte bzw. hoher Schmelzviskositäten vereitelt. So wird in Beispiel 9 ausgeführt, daß sich nach 20 Stunden die Schmelzviskosität bei 245°C auf einen Wert von 350 Poise beläuft. - Wie aber aus dem Stand der Technik bekannt ist und wie beispielsweise der US-PS 3 991 766 [Spalte 6, Zeile 53 bis 55 ] entnommen werden kann, eignen sich für die eingangs angeführten Verwendungszwecke lediglich Polymere mit einer Schmelzviskosität von 400 bis 27 000 Poise (245°C).

Daneben beschreibt die US-PS 3 797 499 bioabsorbierbare orientierte Filamente, die durch Extrusion von Polylactidpolymeren bzw. Copolymeren aus L-Lactid und Glykolid hergestellt worden sind. Die dort beschriebenen Polyester werden zur Aufarbeitung aus Benzol, Toluol oder Xylol mit Hexan umgefällt. Bei der dieser Reinigungsoperation vorgeschalteten Herstellung werden 0,001 bis 2 Gew.-% eines Oxids oder Salzes des Magnesiums, Calciums, Strontiums oder Bariums dem Monomer als Katalysator zugesetzt. Da die Umfällung in der Art, wie sie ausgeführt wird, nicht geeignet ist, diese Bestandteile aus dem Polymerisationsprodukt zu entfernen, ist davon auszugehen, daß das Reaktionsprodukt mindestens 0,001 % oder 10 ppm Katalysator enthält, was der ursprünglich eingesetzten Menge entspricht.

Die US-PS 3 991 766 betrifft schließlich Polyglycolsäure mit vorteilhaften Eigenschaften in Bezug auf die Verwendung als chirurgisches Hilfsmittel oder Arzneimittelwirkstoffträger. In der Beschreibung wird ausgeführt, daß die Polymerisation des Glycolids durch Erhitzen in Gegenwart eines Katalysators oder ohne Katalysator erfolgen kann und ebenso durch Bestrahlung mit Röntgenstrahlen, γ-Strahlen und ß-Strahlen induziert werden kann. Allerdings ist der gesamten Patentschrift kein Hinweis zu entnehmen, wie dies zu erfolgen hätte und welcher Art die aus derartigen Umsetzungen resultierenden Produkte sind.

Wie eigene Ergebnisse der Anmelderin lehren, ist mit derartigen Methoden kein hochmolekulares Produkt, welches zur Herstellung der genannten chirurgischen Hilfsmittel geeignet wäre, erhältlich. Im Einklang damit stehen die Beispiele dieser Patentschrift; dort werden alle Polyester nicht durch Bestrahlung oder Erwärmen hergestellt, sondern unter Zuhilfenahme der Katalyse. Somit resultiert auch aus der in dieser Patentschrift beschriebenen Verfahren ein katalysatorhaltiges Produkt.

Nach dem erfindungsgemäßen Verfahren können resorbierbare Homopolymere und Copolymere hergestellt werden, die praktisch frei von Katalysator sind. Die Polymere sind Homo- und Copolymere auf der Basis von Hydroxycarbonsäuren, die beispielsweise Polymerisate von Glycolid, Lactid, Methylglycolid, Dimethylglycolid, Polymethylglycolid, Diethylglycolid, Dibutylglycolid, Caprolacton, Valerolacton, Decalacton, Propiolacton, Butyrolacton, Pivalolacton Diese Polymere sind in einem organischen Lösungsmittel, gegebenenfalls nach geeigneter Vorbehandlung, z.B. Tempern, löslich.

Als weitere Comonomere sind u.a. die folgenden Verbindungen geeignet:
Milchsäure, Glykolsäure, Pentaerythrit, Sorbit, Adonit, Xylit, Fructose, Epichlorhydrin, Isopropylmorpholin, Isopropylmethylmorpholindion, ß-Propiolsäure, Tetramethylglycolid, β-Butyrolacton, γ-Butyrolacton, Pivalolacton, a-Hydroxyisobuttersäure, α-Hydroxyvaleriansäure, α-Hydroxycapronsäure, α-Hydroxyisocapronsäure, α-Hydroxy-α-ethylbuttersäure, α-Hydroxy-β-methylvaleriansäure, α-Hydroxyheptansäure, α-Hydroxyoctansäure, α-Hydroxydecansäure, α-Hydroxytetradecansäure, α-Hydroxystearinsäure.

Besonders bevorzugt sind katalysatorfreie Homopolymere aus Lactid, katalysatorfreie Copolymere aus verschiedenen Lactiden und katalysatorfreie Copolymere aus Lactiden und Glykolid mit inhärenten Viskositäten zwischen 0.1 und 10 dl/g.

Als Lactid können 1-, d-, meso- oder dl-Lactid oder Gemische davon eingesetzt werden. Bei Copolymeren aus Glycolid und Lactid ist die Löslichkeit des jeweiligen Copolymeren stark vom Glycolidgehalt abhängig.

Weiterhin bevorzugt sind Homopolymere aus l-Lactid mit einer inhärenten Viskosität von 0.1 bis 10 dl/g. Aus der inhärenten Viskosität berechnet sich das entsprechende mittlere Molekulargewicht zu 2 000 bis 1.4 Mio (Lit. W. Dittrich u. R.C. Schulz, Angew. Makro.Chem., 15 (1971) 109-126).

Bevorzugt sind weiterhin Homopolymere aus dl-Lactid mit einer inhärenten Viskosität von 0.1 bis mindestens 5 dl/g, entsprechend mittleren Molekulargewichten von 2 000 bis mindestens 1.5 Mio, berechnet aus der inhärenten Viskosität durch Approximation des Staudinger Indexes nach Solomon und Ciuta (Lit. J. Appl. Polym. Sci, 6,24 (1962), 683-6) und Einsetzen in die Mark-Houwink-Gleichung mit den Parametern K = 6.06 e-4 und a=0.64 (Lit. J. Rak, J.L Ford, Ch. Rostron u. V. Walther, Pharm. Acta Helv., 60, 5-6 (1985), 162-9).

Ferner sind bevorzugt Copolymere aus l-Lactid und Glykolid, wobei wegen der beginnenden Unlöslichkeit der Copolymere mit hohem Glycolidanteil der Glycolidgehalt in der Regel 50 % nicht überschreitet, mit inhärenten Viskositäten von >1.4 bis mindestens 4.5 dl/g. Daraus berechnen sich nach der oben angegebenen Methode und den Mark-Houwink-Parametern K=5.45 E-4 und a=0.73 (Lit. S. Gogolewski u. A.J. Pennings, J. Appl. Polym. Sci., 28, 1045-61) mittlere Molekulargewichte von 50 000 bis mindestens 250 000.

Bevorzugt sind weiterhin Copolymere aus dl-Lactid und Glycolid mit inhärenten Viskositäten von mindestens 1.5 dl/g, entsprechend mittleren Molekulargewichten von mindestens 55 000, berechnet mit den obigen Mark-Houwink-Parametern für Copolymere;
weiterhin Copolymere aus epsilon-Caprolacton mit 1-, dl-Lactid und Glykolid und inhärenten Viskositäten bis mindestens 4 dl/g;
ferner Poly(l-lactid-co-dl-lactid) im Verhältnis 9 : 1, Poly(l-lactid-co-glycolid) im Verhältnis 7 : 3, Poly(dl-Lactid-co-glycolid) im Verhältnis 3 : 1, Poly(dl-lactid-co-glycolid im Verhältnis 1 : 1, Poly(dl-lactid-co-glycolid) im Verhältnis 45:55.

Beispielsweise werden Homopolymere und Copolymere der Milch- und Glykolsäure vorzugsweise durch ringöffnende Polymerisation der cyclischen Diester der Milch- und Glykolsäure, nämlich des Lactids und Glycolids, hergestellt. Auf Grund der Chiralität der Milchsäure können in die ringöffnende Polymerisation die optisch aktiven 1- und d-Lactide, sowie das optisch inaktive meso-Lactid und das Racemat (dl-Lactid) eingesetzt werden.

Die Bedingungen dieser Polymerisation sind bekannt. Für die Katalyse der Polymerisation sind in der Literatur eine Vielzahl von zumeist polyvalente Metallionen enthaltene Verbindungen genannt. Vorzugsweise verwendet man Zinn(II)- oder Zinkverbindungen. Von den Zinnverbindungen wird vorzugsweise das Zinn(II)-di(2-ethylhexanoat) (Zinnoctoat) eingesetzt.

Die Herstellung der Homopolymeren erfolgt in Lösung, Emulsion oder in der Schmelze. Die Copolymere jeder beliebigen Zusammensetzung werden wegen der unterschiedlichen Reaktivität der beiden Comonomeren in Emulsion, vorzugsweise jedoch durch Substanzpolymerisation in der Schmelze hergestellt. Bei der Polymetisation läßt sich durch Variation der Reaktionsparameter Temperatur, Zeit und Katalysatorkonzentration, sowie durch die Zugabe eines oder mehrerer Co-Katalysatoren das jeweils gewünschte Molekulargewicht und die entsprechende Molekulargewichtsverteilung einstellen.

Die nach bekannten Verfahren hergestellten katalysatorhaltigen Polymere werden in einem geeigneten organischen Lösungsmittel oder einem Gemisch aus verschiedenen organischen Lösungsmitteln, welches mit Wasser nicht vollständig mischbar sein darf, gelöst. Im allgemeinen sollte die Konzentration des gelösten Polymeren im Lösungsmittel 10 % nicht übersteigen, da sonst die Lösungen zu viskos werden und eine einwandfreie Aufarbeitung nicht mehr gewährleistet ist. Bevorzugt ist eine Gehalt zwischen 0,5 und 4.0 %, besonders bevorzugt zwischen 0,5 und 2.0 %. Anschließend wird die organische Lösung des Homopolymeren oder Copolymeren in innigen Kontakt mit Wasser einer wäßrigen Lösung einer anorganischen Säure, einer wasserlöslichen organischen Säure oder eines wasserlöslichen Komplexbildners gebracht. Nach einer Kontaktzeit, die ausreichend ist, damit der Katalysator in die wäßrige Phase übergeht, werden die Phasen getrennt und die organische, das katalysatorfreie Polymer enthaltende Phase getrocknet. Aus der organischen Phase wird schließlich das Polymer nach bekannten Verfahren zurückgewonnen.

Als Lösungsmittel sind solche geeignet, die das Homopolymer oder Copolymer lösen, die jedoch nicht mit Wasser mischbar sind oder zumindest in einem bestimmten Mischungsverhältnis ein zweiphasiges System bilden. Bevorzugte Lösungsmittel sind halogenierte Kohlenwasserstoffe, wie. z.B. Methylenchlorid oder Chloroform. Die Löslichkeit der Polymeren ist stark von ihrer Zusammensetzung abhängig und kann durch einfache Versuche ermittelt werden. In einigen Fällen können sich auch Lösungsmittelgemische als besonders geeignet erweisen. In Fällen von schwer löslichen Polymeren, z.B. Copolymere mit einem hohen Anteil von Glycolid ist eine Vorbehandlung, z.B. durch Erhitzen oder Bestrahlen des Polymeren vorteilhaft, um dessen Löslichkeit zu erhöhen. Als anorganische Säuren sind beispielsweise Salzsäure, Schwefelsäure oder Phosphorsäure geeignet, es können aber auch monofuntionelle oder polyfunktionelle wasserlösliche organische Säuren, wie z.B. Essigsäure oder Zitronensäure verwendet werden. Ein geeigneter Komplexbildner ist beispielsweise EDTA (Ethylendiamintetraacetat) in Form der freien Säure oder als Natriumsalz. Die Konzentration der eingesetzten Säuren bzw. Komplexbildner ist nicht kritisch. Zweckmäßigerweise wird eine 0,1 bis 5 %ige, bevorzugt 0.1 bis 1 %ige wäßrige Lösung eingesetzt. Bei der Verwendung stark konzentrierter Säuren kann jedoch nicht ausgeschlossen werden, daß die inhärente Viskosität des Polymeren abnimmt. Verfahren zur Herstellung des innigen Kontakts der beiden nicht mischbaren Phasen sind hinreichend bekannt. Im Labormaßstab erwies sich ein- oder mehrmaliges Ausrühren oder Auschütteln als völlig ausreichend. Im technischen Maßstab wird der Kontakt der beiden nicht mischbaren Phasen zweckmäßigerweise durch Gleichstrom- oder Gegenstromextraktion hergestellt. Entsprechende Verfahren gehören zum Stand der Technik und brauchen nicht näher erläutert werden. Die Extraktion wird solange durchgeführt, bzw. so oft wiederholt, bis sich mit der Methode der Atomabsorption oder einem anderen geeigneten analytischen Verfahren keine Metalle bzw. Metallionen mehr im Homopolymeren oder Copolymeren nachweisen lassen.

Im Anschluß an die Phasentrennung wird die organische Phase getrocknet und das Homopolymer bzw. Copolymer, beispielsweise durch eine geeignete Fällungsmethode, zurückgewonnen und nach dem Trocknen durch die Messung der inhärenten Viskosität charakterisiert. Geeignete Fällungsmethoden sind der Zusatz einer ausreichenden Menge an Fällungsmittel, vorzugsweise eine leichtsiedende Petroletherfraktion. Die Messung der inhärenten Viskosität erfolgt in einem geeigneten Lösungsmittel, z.B. Chloroform oder Hexafluorisopropanol, bei Temperaturen von 25 oder 30 Grad Celsius. Dabei wird unter der inhärenten Viskosität der Quotient aus dem natürlichen Logarithmus der relativen Viskosität und der Konzentration der Meßlösung, angegebene in [g/dl], verstanden. Mit literaturbekannten Beziehungen läßt sich aus der inhärenten Viskosität der Staudinger-Index (Grenzviskosität, intrinsic viscosity) und bei Kenntnis der entsprechenden Parameter mit Hilfe der Mark-Houwink-Gleichung das Molekulargewichtsmittel berechnen. Die Molekulargewichtsverteilung und die Dispersität der Polymeren und Copolymeren wird vorzugsweise durch Ausschlußchromatographie (HPSEC) in Chloroform, Methylenchlorid, Dioxan, THF oder HFIP gemessen. Die Eichung erfolgt dabei nach den bekannten Methoden gegen engverteilte Poly(styrol)-Standards. Es wurde festgestellt, daß durch das erfindungsgemäße Verfahren zur Entfernung des Katalysators die Eigenschaften des Polymeren bzw. Copolymeren (z.B. ausgedrückt durch die inhärente Viskosität) nicht nachteilig verändert werden.

Aufgrund der Tatsache, daß die nach dem erfindungsgemäßen Verfahren hergestellten Polymere praktisch frei von Polymersisationskatalysator sind, eignen sie sich besonders zur Herstellung von Gegenständen, die in menschlichen oder tierischen Körpern resorbiert werden können. Hierzu zählen insbesondere chirurgisches Nahtmaterial, Gegenstände zur osteoysnthese und Arzneimittelwirkstoffträger. Letztere können beispielsweise in Form von Tabletten oder Kapseln, aber auch als implantierbare oder injizierbare Depotformen vorliegen.

Im folgenden werden stellvertretend einige Produkte zur medizinischen Verwendung genannt, die vorteilhaft aus katalysatorfreien Polymeren hergestellt werden können.

Katalysatorfreie, aus resorbierbaren Polymeren hergestellte Produkte:
1. Massive Produkte, formgepreßt oder maschinell bearbeitet:
   Orthopädische Dübel, Klammern, Schrauben und Platten,
   Clips (z.B. für vena cava),
   Krampen,
   Haken, Knöpfe und Schnapper,
   Knochenersatz (z.B. Kieferprothese),
   Nadeln,
   Nichtpermanente Intrauterineinsätze (antispermicid),
   Temporäre Drainage- oder Prüfschläuche oder Kapillaren,
   Chirurgische Instrumente,
   Gefäßimplantate oder -stützmittel,
   Wirbelscheiben,
   Extrakorporale Schläuche für Nieren- und Herz-Lungenmaschinen.
   Langsam aufschließbares Ionenaustauschharz, langsam aufschließbare, Wirkstoffe freigebende Erzeugnisse (Pillen, Pellets),
   verstärkte Knochendübel, Nadeln usw.
   Implantierbare mit Arzneistoffen beladene Pellets, Stifte, Folien und sonstige Formkörper zur kontrollierten Wirkstoff-Freigabe.
2. Faserprodukte, gestrickt oder gewebt, einschließlich Velour
   Brandverbände,
   Bruchkissen,
   Absorbierendes Papier oder Tupfer,
   Medizinalverbände,
   Gesichtsplastiken,
   Gaze, Gewebe, Tuch, Filz oder Schwamm zur Hämostase,
   Gaze-Bandagen,
   Dentalpackungen,
   Nahtmaterial, schließlich Ligaturen.
   Arterientransplantat oder -ersatz Bandagen für Hautoberflächen Brandverbände (in Kombination mit anderen Polymerfilmen)
3. Pulverförmige Produkte, hergestellt durch Sprühtrocknung, Mahlung, Fällung oder Mikroverkapselung.
   Injizierbares oder implantierbares mit Arzneistoffen beladenes Pulver zur kontrollierten und verzögerten Freigabe des Wirkstoffes.
   Mikroporöse Formkörper, Folien, Puder, Granulate zur Beladung mit Wirkstoffen.
4. Verschiedenes
   Flocken oder Puder für Verbrennungen oder Abschürfungen,
   Schaumstoff als absorbierbare Prothese,
   Ersatz für Draht beim Schienen,
   Filmspray für prothetische Elemente und zur Wundversorgung.

### Beispiele

### Beispiel 1 (Vergleichsbeispiel)

16 g Poly(dl-lactid-co-glycolid) im Molverhältnis 1 : 1 mit einem Zinngehalt von 550 ppm und einer inhärenten Viskosität von 0.37 dl/g in Chloroform bei 25 Grad Celsius werden in 80 ml Aceton in der Siedehitze gelöst, filtriert und anschließend in Wasser gefällt. Das Copolymere wird abgesaugt, mit kaltem Wasser nachgewaschen und im Vakuum- oder Umlufttrockenschrank getrocknet. Von der Probe wird der Zinngehalt durch Atomabsorptionsspektrometrie bestimmt und die inhärente Viskosität in Chloroform bei 25 Grad Celsius gemesssen.
- Ausbeute:: 89.8 % des Einatzes
- Inhärente Viskosität: 0.40 dl/g
- Zinngehalt: 415 ppm

### Beispiel 2

Jeweils 100 ml einer 1%igen Chloroformlösung eines Poly(l-lactids) mit einer inhärenten Viskosität von 1.47 dl/g und einem Zinngehalt von 254 ppm werden mit 100 ml einer 0.1 n Salzsäure verschieden lange Zeiten ausgerührt.

Nach der Phasentrennung und der Trocknung der organischen Phase mit Natriumsulfat wird das Poly(l-lactid) durch Zugabe von Petrolether mit einem Siedebereich von 40 - 60 Grad Celcius wieder ausgefällt und im Vakuumtrockenschrank bei ca. 40 Grad Celsius auf Gewichtskonstanz getrocknet.

Der Zinngehalt jeder Probe wird mit Hilfe der Atomabsorption bestimmt.

| Ausrührzeit [Minuten] | Zinn-Gehalt [ppm] |
|---|---|
| 0 | 254 |
| 5 | 29 |
| 15 | <2 |

### Beispiel 3

Jeweils 100 ml einer 1%igen Chloroformlösung eines Poly(l-lactids) mit einer inhärenten Viskosität von 1.47 dl/g in Chloroform bei 25 Grad Celcius und einem Zinngehalt von 254 ppm werden einmal, zweimal und dreimal mit jeweils 100 ml einer 0.1 n Salzsäure 5 Minuten ausgerührt. Die Phasen werden getrennt, die wäßrigen Phasen werden verworfen und die organischen Phasen jeweils mit Natriumsulfat getrocknet. Das Poly(l-lactid) wird durch Zugabe von Petrolether mit einem Siedebereich von 40- 60 Grad Celsius wieder ausgefällt und im Vakuumtrockenschrank bei ca. 40 Grad Celsius auf Gewichtskonstanz getrocknet. Die Ausbeute beträgt durchweg 85 % des Einsatzes.

Der Zinngehalt jeder Probe wird mit Hilfe der Atomabsorption bestimmt.

| Ausrührung [Anzahl] | Zinn-Gehalt [ppm] |
|---|---|
| 0 | 254 |
| 1 | 29 |
| 2 | 2 |
| 3 | <2 |

### Beispiel 4

100 ml einer 1%igen Lösung eines Poly(l-lactids) in Chloroform mit einer inhärenten Viskosität von 1.47 dl/g und einem Zinngehalt von 254 ppm werden jeweils mit 100 ml Wasser, einer 1 %igen wässrigen Lösung einer organischen Säure, einer Mineralsäure oder eines Komplexbildners 15 Minuten ausgerührt. Man läßt 10 Minuten absitzen und trennt dann die Phasen. Die wäßrige Phase wird verworfen. Nach der Phasentrennung und der Trocknung der organischen Phase mit Natriumsulfat wird das Poly(l-lactid) durch Zugabe von Petrolether mit einem Siedebereich von 40 -60 Grad Celsius wieder ausgefällt und im Vakuumtrockenschrank bei ca. 40 Gad Celsius auf Gewichtskonstanz getrocknet.

| Wäßrige Lösung | Ausbeute [%d.Eins.] | Sn-Gehalt [ppm] |
|---|---|---|
| Wasser | 90 | < 2 |
| Schwefelsäure | 94 | < 2 |
| Salzsäure | 91 | < 2 |
| Essigsäure | 92 | < 2 |
| Phosphorsäure | 88 | < 2 |
| Zitronensäure | 90 | < 2 |
| EDTA | 95 | < 2 |

### Beispiel 5

Jeweils 100 ml einer 1%igen Lösung eines Poly(dl-lactid-co-glycolid) in Chloroform mit einem Zinngehalt von 550 ppm werden mit 100 ml einer 1%iqen Salzsäurelösung 15 Minuten ausgerührt.

Nach der Phasentrennung und der Trocknung der organischen Phase mit Natriumsulfat wird das Copolymerisat wieder ausgefällt und im Vakuumtrockenschrank bei ca. 40 Grad Celsius auf Gewichtskonstanz getrocknet.

| Molverhältnis | Ausbeute [%d.Eins.] | Sn-Gehalt [ppm] |
|---|---|---|
| 50 : 50 | 99 | < 2 |
| 75 : 25 | 96 | < 2 |

## Patentansprüche

1. Verfahren zur Herstellung katalysatorfreier resorbierbarer Homopolymere oder Copolymere auf der Basis von Hydroxycarbonsäuren, dadurch gekennzeichnet, daß man ein katalysatorhaltiges Polymer in einem organischen nicht mischbaren Lösungsmittel löst, in innigen Kontakt mit Wasser oder mit einer wäßrigen Phase, die eine nichtorganische Säure, eine wasserlösliche organische Säure oder einen wasserlöslichen Komplexbildner enthält, bringt, die Extraktion solange durchführt, bzw. so of wiederholt, bis sich mit der Methode der Atomabsorption oder einem anderen geeigneten analytischen Verfahren keine Metalle bzw. Metallionen mehr (weniger als 2 ppm) in Homopolymeren oder Copolymeren nachweisen lassen, die organische Phase abtrennt und daraus das Polymer nach an sich bekannten Verfahren isoliert.

2. Verfahren nach Anspruch 1, dadurch gekennzeichnet, daß die Säure oder der Komplexbilder in der wäßrigen Phase in einer Konzentration von 0.1 bis 5 % vorliegt.

3. Verfahren nach Anspruch 1 oder 2, dadurch gekennzeichnet, daß die Konzentration des gelösten Polymers in dem Lösungsmittel 10 % nicht überschreitet.

## Claims

1. Process for producing catalyst-free resorbable homopolymers or copolymers based on hydroxycarboxylic acids, characterised in that a catalyst-containing polymer is dissolved in an organic solvent which is immiscible with water, then brought into intimate contact with water or with an aqueous phase which contains an inorganic acid, a water-soluble organic acid or a water-soluble complexing agent extraction is continued or repeated until no more metals or metal ions (less than 2 ppm) can be detected in the homopolymer or copolymer using the atomic absorption method or another suitable analytical method, the organic phase is separated off and the polymer is isolated therefrom by methods known per se.

2. Process according to claim 1, characterised in that the acid or the complexing agent is present in the aqueous phase in a concentration of from 0.1 to 5%.

3. Process according to claim 1 or 2, characterised in that the concentration of the dissolved polymer in the solvent does not exceed 10%.

## Revendications

1. Procédé de préparation d'homopolymères ou de copolymères résorbables exempts de catalyseur, à base d'acides hydroxycarboxyliques, caractérisé en ce que l'on dissout un polymère contenant un catalyseur dans un solvant organique non miscible à l'eau, on le met en contact intime avec de l'eau ou avec une phase aqueuse qui contient un acide inorganique, un acide organique hydrosoluble ou un complexant hydrosoluble, on opère ou répète l'extraction jusqu'à ce qu'il ne soit plus possible de mettre en évidence des métaux ou des ions métalliques (moins de 2 ppm) dans l'homopolymère ou le copolymère avec la méthode d'absorption atomique ou avec un autre procédé analytique approprié, on sépare la phase organique et on isole à partir de celle-ci le polymère selon des procédés connus en soi.

2. Procédé selon la revendication 1, caractérisé en ce que l'acide ou le complexant est présent dans la phase aqueuse en une concentration de 0,1 à 5%.

3. Procédé selon la revendication 1 ou 2, caractérisé en ce que la concentration du polymère dissous dans le solvant ne dépasse pas 10%.
